Europäisches Patentamt

European Patent Office

Office européen des brevets

(11) Publication number: **0 281 530 B1**

## EUROPEAN PATENT SPECIFICATION

(45) Date of publication of patent specification: **18.05.94**

(51) Int. Cl.⁵: **C12N 15/75**, C12N 1/21, C12P 21/00, C12N 15/70, //(C12N1/21,C12R1:19), (C12N1/21,C12R1:125)

(21) Application number: **88830078.7**

(22) Date of filing: **01.03.88**

(54) **Recombinant plasmids useable for the expression of heterologous proteins in bacillus.**

(30) Priority: **03.03.87 IT 1955187**

(43) Date of publication of application:
**07.09.88 Bulletin  88/36**

(45) Publication of the grant of the patent:
**18.05.94 Bulletin  94/20**

(84) Designated Contracting States:
**AT BE CH DE ES FR GB GR LI LU NL SE**

(56) References cited:
**EP-A- 0 120 629**
**EP-A- 0 218 468**
**EP-A- 0 232 229**

**Chemical Abstracts, vol. 106, no.11, March 16, 1987, Columbus, Ohio, USA; M.J. Keith et al "Pertussis toxin gene cloning and expression of protective antigen" page 163, abstract no.**

(73) Proprietor: **ENIRICERCHE S.p.A.**
**Corso Venezia 16**
**I-20121 Milan(IT)**

Proprietor: **SCLAVO S.p.A.**
**Via Fiorentina 1**
**I-53100 Siena(IT)**

(72) Inventor: **Velati Bellini, Ada**
**Via Libertà 17/1**
**I-27100 Pavia(IT)**
Inventor: **Toma, Salvatore**
**Viale Caterina da Forli, 5**
**I-20146 Milan(IT)**
Inventor: **Grandi, Guido**
**Nuova Strada, 4**
**I-20090 Segrate S. Felice Milan(IT)**
Inventor: **Colletti, Elisabetta**
**Via Catania, 3**
**I-20133 Milan(IT)**
Inventor: **Riboli, Barbara**
**Via Borgo Spera, 1**
**I-26100 Cremona(IT)**
Inventor: **Cosmina, Paola**
**Viale Misurata, 14**
**I-20146 Milan(IT)**

Inventor: **Tognoni, Angelo**
**Via delle Rose, 3**
**I-20090 Pieve Emanuele Milan(IT)**
Inventor: **Pedroni, Paola**
**Via Plinio, 48**
**I-20129 Milan(IT)**
Inventor: **Rappuoli, Rino**
**Via Calamandrei, 35**
**I-53010 Quercegrossa Siena(IT)**

(74) Representative: **Hallybone, Huw George et al**
**CARPMAELS AND RANSFQRD**
**43 Bloomsbury Square**
**London WC1A 2RA (GB)**

## Description

The present invention concerns a bifunctional vector for the cloning of a DNA sequence which codes for a heterologous protein; recombinant plasmids expressed in Bacillus containing the DNA sequence; host micro-organisms transformed by the recombinant plasmids and a method for the preparation of the proteins.

More particularly, the present invention relates to a method and means for the production of the mature subunits of the pertussis toxin, usable for the preparation of anti-pertussis vaccines, and of detection systems for the diagnosis of pertussis.

Pertussis is an infection of the respiratory tract caused by Bordetella pertussis (B. pertussis), a Gram-negative coccobacillus which is transmitted directly during the catarrhal or convulsive stage by aerogenic means from the sick person to a susceptible healthy individual.

Pertussis can cause respiratory complications, nerve-damage and high mortality, above all in children and in newborn babies having no maternal pertussis antibodies.

The clinical course of pertussis includes four stages: incubation, the catarrhal stage, the paroxysmal stage and the convalescent stage.

During the first two stages there are symptoms similar to a common cold and B.pertussis can easily be isolated from the patients.

During the paroxysmal stage, characterised by the symptoms particular to pertussis, the bacterium can be isolated only in 50% of cases.

In the convalescent stage, it is no longer possible to isolate B. pertussis from the nasopharynx, but symptoms of pertussis persist in the patients.

From this picture, it is clear that the patient presents his most virulent clinical signs after the bacteria have disappeared. However, pertussis is not due to invasion of the respiratory tract by the B. pertussis but to a toxic state, induced by the bacteria and which persists even after they have disappeared.

The change of B. pertussis from stage I (virulent) to stage III (non-virulent) is accompanied by a loss of capacity to synthesise certain substances such as: pertussis toxin (PT), haemolysin (Hly) adenylcyclase (Adc) and dermonecrotic toxin (Dnt).

Recently Weiss A.A. et al. (Inf. Immun. 42, 33 (1983); J. Inf. Dis. 150, 219 (1984)) have observed that not all these substances contribute equally to the virulence of B. pertussis and have found that the pertussis toxin represents the major factor in the virulence of this bacterium.

Pertussis toxin, a protein with a molecular weight of 100,000 daltons, is produced and released into the extracellular environment by B. pertussis during stage I.

The pertussis toxin, like other toxins, is constituted by two different fragments: A and B.

The toxic A fragment comprises a single polypeptide S1 (S1 subunit) with a molecular weight of approximately 28,000 daltons, which is able to bind an ADP-ribose group to a GI protein involved in the transmission of signals from the outside to the inside of the cell.

The B fragment comprises 5 polypeptides S2, S3, S4, and S5 (S2, S3, S4 and S5 subunits) with molecular weights of 23,000, 22,000, 12,000 and 9,000 daltons respectively, arranged as 2 dimers S2 + S4 and S3 + S4 and a monomer S5.

The B fragment binds to eukaryotic cell membrane receptors facilitating entry of the S1 subunit into the cells.

An antipertussis vaccine is currently used which, whilst conferring permanent immunity, has numerous disadvantages.

The vaccine is constituted by virulent bacteria (stage I) treated at 56° Centigrade for 30 minutes to remove a toxin which is labile to heat (dermonecrotic toxin), and killed with merthiolate.

Since the bacteria are not subjected to any detoxification treatment, any toxic substances which can withstand a temperature of 56° Centigrade will be included in the vaccine.

The presence of these toxic substances, and particularly PT, causes side effects which range from a simple rash to permanent neurological damage and/or death.

All this means that, in the last ten years, the use of the vaccine has been drastically reduced with a consequent breaking out of pertussis cases again.

The preparation of anti-pertussis vaccine, constituted essentially by fibrous haemagglutinin (FHA) and pertussis toxin detoxified with formaldehyde, is described by Sato Y. et al. in Lancet Jan. 21, 122 (1984).

This vaccine is not yet satisfactory in that it induces side effects, although less than those of the conventional vaccine, and is not obtainable in a pure and reproducible form.

As a result of all this, there is a need to have alternative sources available for the preparation of an effective anti-pertussis vaccine, which can be produced on a large scale and which has none of the disadvantages described above.

Thus, for example, recent developments in the field of genetic engineering have enabled the construction of micro-organisms which can produce heterogolous proteins usable in therapy. In particular, ITALIAN patent application No. A/86 19208 describes and claims the sequencing and cloning of genes which code for the subunits of pertussis toxin and a method for the preparation of these subunits which comprises the culture in a suitable culture medium of transformed Escherichia coli (E.coli) cells. However, the use of this method produces fused proteins, that is, proteins constituted by the mature subunits of the pertussis toxin and by the enzyme polymerase. These proteins could, therefore, require hydrolytic treatment before being used in the preparation of antipertussis vaccines.

Furthermore, the use of E.coli, a bacterium which is pathogenic in man, as the host micro-organism, makes the method itself quite onerous, thus complicating its execution, particularly at the stage of separation and recovery of the proteins.

These disadvantages may be overcome by the use of Bacillus, a non-pathogenic bacterium which does not produce endotoxins, and of a cloning vector which is stable in Bacillus and which enables the expression of the mature subunits of pertussis toxin.

An aspect of the present invention is therefore constituted by a bifunctional vector useful for the cloning of DNA sequences which code for the mature subunits of pertussis toxin in Bacillus.

Another aspect of the present invention is constituted by recombinant plasmids for expression in Bacillus, obtained by the cloning in the EcoRI and HindIII restriction sites of the cloning vector, of the DNA sequences which code for the mature subunits of pertussis toxin.

A further aspect of the present invention is constituted by a method for the preparation of the mature subunits of pertussis toxin by means of the culture of Bacillus cells transformed by the recombinant plasmids.

Further aspects of the present invention will become clear from the description and examples which follow.

In particular, the cloning vector according to the present invention, known as pSM 214, contains the functional origins of replication of pUB110 and pBR322, which enable the replication, in B.subtilis and in E.coli, of Km, Bla and Cat genes which code respectively for resistance to the antibiotics kanamycin, ampicillin and chloramphenicol, and a strong artifical promoter which directs the transcription of a dicystronic messenger RNA (mRNA) including the Bla and Cat gene sequences. The formation of such mRNA is particularly advantageous in that it enables the stabilisation of strong promoters in the plasmids by selection of the transformants on chloramphenicol. In particular, the cloning vector according to the present invention is obtained by means of a method which comprises:

a) isolation from the plasmid pC194, by means of digestion with the restriction enzymes MboI and HpaII, of a DNA fragment of 1032 base pairs (bp) formed by the 820-bp gene which codes for chloramphenicol-acteyl-transferase (Cat), and of the 212-bp sequence of its promotor;

b) isolation of the 820-bp gene from the 1032-bp fragment by means of partial digestion with the restriction enzyme HinfI, and construction of blunt ends on the 820-bp fragment with the Klenow DNA polymerase enzyme;

c) binding of the 820-bp fragment to the linearised plasmid pSM 191 by means of digestion with the restriction enzyme HincII and isolation of the plasmid pSM 213 thus obtained;

d) isolation from the plasmid pSM 213, by means of sequential digestion with restriction enzymes BamHI and Hind III, of a DNA fragment of approximately 1000 bp, containing the gene which codes for Cat and the sequence of the to terminator of the lambda phage of E.coli;

e) the binding of the 1000-bp fragment to the 6300-bp DNA fragment isolated from the plasmid pSM 208-A after digestion of the latter with the restriction enzymes XhoI and HindIII and finally;

f) isolation of the 7300-bp cloning vector pSM 214 from transformed strains of B. subtilis .

In accordance with the present invention, the digestion of the plasmid DNA is carried out, according to generally known techniques, by suspension of the DNA in buffer solutions in the presence of one or more suitable restriction enzymes, and maintenance of the suspension thus obtained at the temperature and for the period of time needed to complete the digestion reaction.

On completion of the reaction, the fragment and/or the fragments of DNA obtained are separated by means of electrophoresis on polyacrylamide gel by the application of a potential difference of from 80 to 150 volts and, finally, the fragment with the desired sequence is isolated by means of electro-elution according to the method described by Maxam and Gilbert (Methods in Enzymology (1980), vol. 45, 499-560). The ligation reaction is carried out in a buffer solution in the presence of T4 DNA ligase enzyme, at a temperature of from 14 ° Centigrade to 25 ° Centigrade for a period of from 10 to 20 hours.

On completion of the ligation reaction, the hybrid plasmids obtained are isolated by the transformation of E.coli K12 cells, made competent by the method of Maniatis et al. (Molecular Cloning; a Laboratory Manual, 1982), with the ligase mixture and by selection of the transformants on plates of a medium of lactose agar broth (DIFCO) for resistance to ampicillin and/or Chloramphenicol.

Examples of E.coli suitable for the purpose are E.coli JM 83 (pro, ara, Δ lacpro, strA, Φ 80d, lacZ, M15), E.coli HB101 (F⁻, hsdS20, ($r_B^-$ $m_B^-$) recA13, ara-14, pro A2, lacY1, galK2, rpsL20(Sm$^R$), Xyl-5, mtl-1, sup E44λ⁻) and E.coli JM101 (lac, pro, supE, thi F' proAB, lac⁻$^q$).

According to the present invention, in step c) of the method given above, the plasmid psM191 is constructed by the insertion into the XbaI restriction site of pUC 13 described by Vieira J. & Messing J. (-(1982) (Gene 19, 259) of the synthetic XbaI-XbaI fragment whose sequence is

```
5'CTAGAGACTCCTGTTGATAGATCCAGTAATGACCTCAGAACTCCATCTGGATTT
3'TCTGAGGACAACTATCTAGGTCATTACTGGAGTCTTGAGGTAGACCTAAA

GTTCAGAACGCTCGGTTGCCGCCGGGCGTTTTTATTGGTGAGAATCT 3'
CAAGTCTTGCGAGCCAACGGCGGCCCGCAAAAATAACCACTCTTAGAGATC 5'
```

This fragment, synthesised according to generally-known techniques with the use of a System One DNA Synthesizer (Beckman), represents the sequence of the to terminator of the lambda phage of E.coli, which is particularly effective in directing termination of the transcription of the DNA situated upstream of this terminator.

In the plasmid pSM191, the synthesised fragment is oriented in the same sense as the Lac promotor of pUC13 and is able to stop the transcription which takes place starting from this promotor. According to the present invention, in step e) of the method given above, the plasmid pSM 208-A is obtained by digestion of the plasmid pSM 208, described in copending ITALIAN application No. A/86 2265, with XbaI and EcoRI restriction enzymes, and subsequent binding of the pSM 208 7300-bp fragment to a 74-base fragment of synthesised DNA, whose sequence is:

```
                          -35                      -10
    5'   CTAGAAAAATTTATTTGCTTTCAGGAAATTTTTTATGTATAATAG
         3' TTTTTAAATAAACGAAAGTCCTTTAAAAAATACATATTATC

                          RBS
    ATTCATAAATTTGAGAGCTCAAAGGAGGAATTCTTATG 3'
    TAAGTATTTAAACTCTCGAGTTTCCTCCTTAAGAATACTTAA 5'
                                        EcoRI
```

This artificial fragment contains the sequence of a promotor which directs the transcription of the messenger RNA very efficiently and is positioned, in the plasmid pSM 208-A, upstream of the β-lactamase gene (Bla) which confers resistance to ampicillin.

Subsequent digestion of the plasmid with the restriction enzymes XhoI and HindIII generates a 6300-bp fragment containing the artificial promotor and the β-lactamase gene.

Introduction, in exact orientation, of the 1000-bp fragment containing the Cat gene, without its own promotor, and the to terminator of the lambda phage, downstream of the β-lactamase gene of the 6300-bp fragment confers the required properties on the resultant pSM 214 vector, that is, resistance to the three antibiotics and the capacity to transcribe dicystronic mRNA which includes the sequences of the Bla and Cat genes.

The vector thus obtained is particularly suitable for the construction of plasmids for the expression of heterologous proteins in B.subtilis and in E.coli. In fact, removal of the β-lactamase gene from pSM 214, with the restriction enzymes EcoRI and HindIII, and the subsequent introduction of a heterologous gene into the same restriction sites enables the construction of recombinant plasmids in which transcription of the gene is ensured by the presence of a single promotor, the selection being effected on plates containing chloramphenicol.

In particular, according to the present invention, the vector pSM 214 is used for construction of recombinant plasmids pSM 226, pSM 228, pSM 244 and pSM 243 containing the genes which code for the mature subunits of pertussis toxin S2, S3, S4 and S5 respectively.

According to the present invention, and in order to cause expression of the mature subunits of pertussis toxin, that is, subunits equivalent to those occurring naturally, plus a methionine in the aminoterminal position, the signal sequence for the secretion of the protein is removed from the complete gene which codes for the precursor of the subunit and thus the DNA sequence which codes only for the mature portion of the subunit is cloned in the vector pSM 214.

In particular, the complete genes which code for the precursors of the individual subunits of pertussis toxin are isolated from the plasmid PT 110, which has previously been digested with suitable restriction enzymes and, by means of the construction of intermediate plasmids, the DNA sequences which code only for the mature part of the subunits are isolated.

The sequences are then cloned in the EcoRI and HindIII restriction sites of pSM 214 so that the codon of the first amino acid of the mature sequence is juxtaposed to the ATG of the translation initiation codon.

In order to obtain exact insertion of the DNA sequence for mature S2 in pSM 214, a new, simple and effective method of mutagenesis in vitro has been developed.

This method, which falls within the scope of the present invention, requires neither the preparation of a single-stranded DNA, nor polymerisation in vitro and a ligation reaction.

In general, according to this method, the plasmid DNA (A DNA) is linearised with a restriction enzyme (RE I) which cuts near the region to be modified. This region, which may be represented by one or more nucleotide bases, is removed by means of digestion with Ba131 or ExoIII + S1 and the 5' terminals are exposed by means of treatment with ExoIII.

A second restriction enzyme (RE II) is used to cut the molecule into two fragments B and C and the DNA fragment (B DNA), from which the region to be modified has been removed, is purified.

The A DNA linearised with RE I is treated separately with Exo to expose the 3' end and subsequently digested with RE II and the C fragment (C DNA) is purified.

If RE I generates projecting 3' ends, the treatment with Exo is not necessary. An oligonucleotide (D DNA) is synthesised simultaneously so that its sequence is complementary, apart from the projecting terminals of B DNA and C DNA, and re-forms a sequence which is identical to the original one except in the region to be modified.

The B, C and D DNAs are then mixed in ligase buffers in the presence of T4 DNA ligase, and the entire mixture is used for transforming E.coli cells.

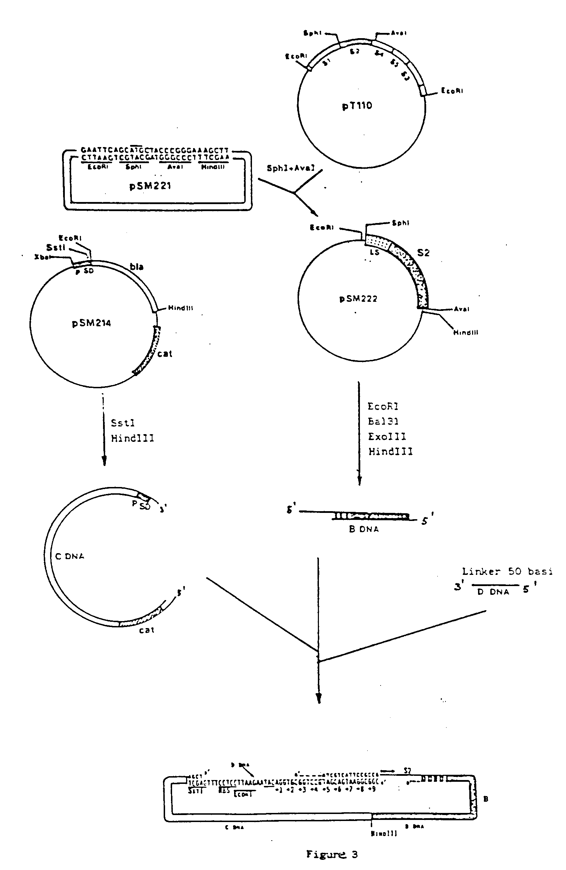

In particular, with reference to Figure 3, the intermediate plasmid pSM 222 is linearised with the restriction enzyme SstI and treated with Bal 31 to remove, from the aminoterminal region of S2, the nucleotides which code for the signal sequence responsible for secretion.

Subsequent treatment of the DNA with ExoIII causes exposure of the 5' ends.

Digestion of the DNA with the restriction enzyme HindIII enables the 6500-bp fragment containing the gene which codes only for the mature S2 to be isolated.

In order to reconstruct the complete sequence of the S2 mature subunit and enable its subsequent cloning in pSM 214, a single-stranded oligonucleotide of 50 bases is synthesised, which is capable of binding at one end to the aminoterminal region of mature S2, and thus recreating the complete information for the first 9 aminoacids plus methionine, and at the other end to the control sequence of pSM 214.

The recombinant plasmids pSM 226, pSM 228, pSM 244 and pSM 243, obtained by cloning the DNA coding sequences for the mature pertussis toxin subunits S2, S3, S4 and S5 respectively, in pSM 214 are used for transforming B. subtilis cells.

In particular there are used cells of SMS118 B. subtilis (leu, pyr D1, npr⁻ spr⁻), a neutral protease minus and serinic protease minus strain isolated in our laboratory, made competent by the method described by Contente and Dubnau (Mol. Gen. Genet. (1979), 167, 251-258), the transformants being selected for chloramphenicol resistance on a TBAB (DIFCO) medium.

The B. subtilis SMS 118 (pSM 226), SMS 118 (pSM 228), SMS 118 (pSM 244), SMS 118 (pSM 243) and SMS 118 (pSM 214) strains were deposited on 12.2.1987 at the American Type Culture Collection in accordance with the Budapest Treaty, respectively, with the following codes: (ATCC 67321), (ATCC 67322), (ATCC 67324), (ATCC 67323), (ATCC 67320).

According to the present invention, and in order to confirm expression of the mature pertussis toxin subunits, the SMS118 B. subtilis cells transformed by the recombinant plasmids pSM 226, 228, 244 and 243 respectively, were grown in a liquid VY (DIFCO) medium in the presence of chloramphenicol at a temperature of from 25 to 40° Centigrade and subsequently lysed by one of the generally-known techniques.

Analysis of the lysed cells by means of dyeing with Coomassie blue, according to Laemmli's method (Nature, 1970 277, 680) showed that the protein expressed by these strains of B. subtilis have molecular weights of 23,000, 22,000, 12,000 and 10,000 daltons respectively and co-migrate with the mature subunits S2, S3, S4 and S5 of the natural toxin.

Analysis by means of transfer on to nitrocellulose according to Towbin's method showed that the proteins react specifically with anti-S2, S3, S4 and S5 antibodies, thus confirming their immunological identity.

The subunits may thus be used for the preparation of anti-pertussis vaccines and in systems for the diagnosis of pertussis.

Brief description of the terms used:

Restriction enzymes: are hydrolytic enzymes capable of cutting a DNA molecule at specific sites, the restriction sites.

Expression: this is the mechanism by which an organism is capable of synthesising a protein coded by a particular gene. In this case it is said that the gene is expressed by the micro-organism.

Recombinant plasmids: are circular-shaped extrachromosomal DNA molecules containing a fragment of heterologous DNA.

Promotor: this is a specific region of the DNA molecule in which the RNA polymerase enzyme initiates transcription. In the promotor there is a recognition site and a binding site for the enzyme.

Terminator: this is a region of the DNA molecule at which transcription terminates.

Transcription: this is the process of transferring genetic information from the DNA to messenger RNA (mRNA).

Translation: this is the polymerisation of amino acids to form proteins on the basis of genetic information coded by the mRNA.

Cloning Vector: is a DNA molecule which contains all the genetic information which enables replication in a host micro-organism and can be used for introduction of heterologous DNA into the micro-organism.

Examples of cloning vectors are natural plasmids or plasmids constructed by joining two or more sequences of DNA from different origins.

The experimental examples below are illustrative of the invention and are not limiting.

Brief description of the drawings.

Figure 1a - shows the restriction map of the plasmid pSM 191 obtained by insertion of the artificial sequence of the to terminator of the lambda phage (to) of E.coli into the XbaI restriction site of pUC13.

Figure 1b - shows the plasmid pSM 208-A obtained by substitution of the sequence of the artificial promotor for the EcoRI-XbaI fragment of pSM 208.

Figure 2 - shows the construction of the cloning vector pSM 214 and the restriction map of the intermediate plasmid pSM 213.

Figure 3 - shows the construction of the recombinant plasmid pSM 226.

Figures 4 and 5 - show the construction of the recombinant plasmid pSM 228.

Figures 6 and 7 - show the construction of the recombinant plasmid pSM 244.

Figures 8 and 9 - show the construction of the recombinant plasmid pSM 243,

Figure 10A - 15% Polyacrylamide SDS gel, where the total proteins extracted from B.subtilis SMS 118 (pSM 226), (pSM 228), (pSM 244) and (pSM 243) appear at 1, 2, 3 and 4 respectively.

Figure 10B - immuno-blot from 15% polyacrylamide SDS gel of the total proteins extracted from B.subtilis SMS 118 (pSM 226), (pSM 228), (pSM 244), and (pSM 243) respectively with anti- S2, S3, S4 and S5 rabbit antibodies and goat anti-rabbit IgG antibodies combined with peroxidase.

EXAMPLE 1

Construction of the cloning vector pSM 214

A) Isolation of the gene which codes for the enzyme Chloramphenicol-Acetyl-Transferase (CAT).

10 $\mu$g of pC194 DNA (Horinouchi and Weisblum, J. Bacteriol. 1982, 150, 815-825) were digested with 30 units (U) of each of the MboI (Biolabs) and HpaII (Boehringer) restriction enzymes, in 100 $\mu$l of a buffer solution containing 10 mM pH 7.5 Tris-HCl, 10 mM MgCl$_2$ and 50 mM NaCl, at 37°C for 1 hour.

The enzyme reaction was then stopped by extraction of the reaction mixture with an equal volume of phenol-chloroform (1:1, v/v) and chloroform-isoamyl alcohol (24:1, v/v).

The plasmid DNA was precipitated by the addition of 2.5 volumes of ethanol and sodium acetate to the reaction mixture to give a final concentration of 0.3M, the resultant mixture being kept at a temperature of -80°C for 15 minutes.

The plasmid DNA precipitated was separated from the reaction mixture by centrifuging in an Eppendorf centrifuge model 5450 at 4°C for 15 minutes and then dried under vacuum.

Analysis of the DNA thus isolated on 5% polyacrylamide gel, as expected, showed three bands corresponding to 1032, 975 and 905 base pairs (bp) respectively which were difficult to resolve from each other. In order to isolate the 1302 bp band containing the gene sequence necessary for construction of the vector of the present invention, after precipitation and isolation, the plasmid DNA was re-suspended in 100 $\mu$l of a buffer solution containing 10 mM pH8 Tris-HCl and 10 mM MgCl$_2$ and digested with 30 U of the restriction enzyme ClaI (BRL) at 37°C for one hour.

The enzyme reaction was then stopped by keeping the reaction mixture at 65°C for 10 minutes.

By means of the procedure described above, only the DNA fragments corresponding to the 975 and 905 bp bands were digested by the enzyme ClaI, as they possess the recognition site for this enzyme, whilst the fragment corresponding to the 1032 bp band remained intact.

The plasmid DNA was then precipitated from the digestion mixture, separated and loaded onto a 6% polyacrylamide gel. After 3 hours running at 120 volts, the 1032 bp fragment was eluted from the gel by the method described by Maxam and Gilbert (Methods in Enzymology (1980), Vol. 65, 499-560).

In order to separate the gene which codes for CAT (820 bp) from its promotor sequence, contained in the HpaII-HinfI fragment of 212 bp, the 1032 bp fragment was re-suspended in 80 $\mu$l of a buffer solution containing 6mM pH 7.5 Tris-HCl, 100mM NaCl, 6mM MgCl$_2$, and 6mM of mercaptoethanol and partially digested with 2 U of HinfI at 37°C for 15 minutes.

The reaction was then stopped by extraction of the reaction mixture with an equal volume of phenol-chloroform (1:1, v/v) and chloroform-isoamyl alcohol (24:1, v/v) and the DNA precipitated after addition of sodium acetate (final concentration 0.3M) and 2.5 volumes of ethanol at -80°C for 15 minutes.

The DNA was then separated by centrifuging, dried under vacuum and finally re-suspended in 200 $\mu$l of a buffer solution containing 50 mM pH 7.2 Tris-HCl, 10 mM MgSO$_4$, 0.1 mM dithiothreitol (DTT), 50 $\mu$g/ml of bovine serum albumin (BSA) and 80 mM each of deoxyguanosine triphosphate (dGTP), deoxycytidine triphosphate (dCTP), deoxythymidine triphosphate (dTTP), and deoxyadenine triphosphate (dATP), and 8 U of DNA polymerase (Klenow large fragment).

The resultant mixture was kept at ambient temperature (20-25°C) for 30 minutes and was then deactivated by the addition of 25 mM of ethylenediaminotetracetic acid (EDTA). The proteins were extracted with phenol-chloroform and chloroform-isoamyl alcohol. The DNA was precipitated from the aqueous phase with sodium acetate (final concentration 0.3 M) and 2.5 volumes of ethanol at -80°C for 15 minutes, separated by centrifuging and dried under vacuum.

The DNA thus obtained was re-suspended in 80 $\mu$l of TE (10 mM pH8 Tris-Cl 1 mM EDTA), loaded onto a 7% polyacrylamide gel and run at 120 volts for three hours.

The DNA fragment corresponding to the 820 bp band was eluted by Maxam and Gilbert's method.

### B) Construction of the plasmid pSM 191

10 $\mu$g of plasmid pUC13 (Boeringer) DNA were suspended in 100 $\mu$l of a buffer solution containing 10 mM pH 7.5 Tris-HCl, 10 mM MgCl$_2$, and 50 mM NaCl and digested with 30 U of XbaI (BRL) restriction enzyme at 37°C for 15 minutes. The enzyme reaction was stopped by keeping the reaction mixture at 65°C for 10 minutes. The plasmid DNA was precipitated by the addition of 2.5 volumes of ethanol and 0.3 M of sodium acetate and then separated by centrifuging. 1 $\mu$g of the DNA thus obtained was then re-suspended in 10 $\mu$l of a buffer solution containing 50 mM pH 7.4 Tris-Cl, 10 mM MgCl$_2$, 10 mM DTT, 1 mM spermidine and 1 mM ATP and ligated, in the presence of 2U of T4 DNA ligase, at 37°C, to the artificial fragment whose sequence is:

8

5'CTAGAGACTCCTGTTGATAGATCCAGTAATGACCTCAGAACTCCATCTGGATTT

3'   TCTGAGGACAACTATCTAGGTCATTACTGGAGTCTTGAGGTAGACCTAAA

GTTCAGAACGCTCGGTTGCCGCCGGGCGTTTTTATTGGTGAGAATCT  3'

CAAGTCTTGCGAGCCAACGGCGGCCCGCAAAAATAACCACTCTTAGAGATC  5'

This fragment, synthesised according to generally known techniques, represents the to terminator of the lambda phage of E.coli, which is particularly efficient in directing the termination of transcription.

The whole ligase mixture was used to transform E.coli HB101 cells made competent by the method described by Maniatis et al. (Molecular Cloning: a Laboratory Manual 1982), the transformants being selected on plates of LB agar (10 g/l of Bacto Triptone, 10 g/l of NaCl, 5 g/l of Yeast extract pH 7.5) (DIFCO) and 100 $\mu$g/ml of ampicillin.

The plasmid DNA was extracted from the ampicillin-resistant (Amp[R]) colonies thus obtained by the method described by Rodriguez and Tait (Recombinant DNA Techniques, p. 50-51; Addison-Wesley Publishing). On restriction analysis, one of these plasmids showed the same insertion and orientation of the recombinant sequence as the pUC13 Lac promotor.

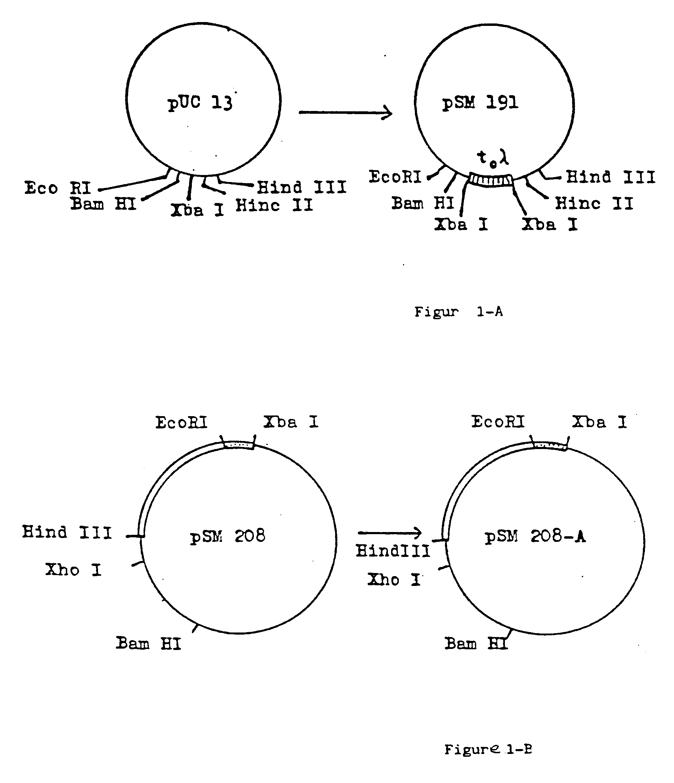

The plasmid thus obtained was designated by the abbreviation pSM 191 and its map is given in Figure 1A.

### C) Insertion of the CAT gene into pSM191

4 $\mu$g of the plasmid pSM 191 were suspended in 20 $\mu$l of a buffer solution containing 10 mM pH 7.5 Tris-HCl, 7 mM MgCl$_2$, 60 mM NaCl and 1 mM of DTT and digested at 37°C for 30 minutes with the restriction enzyme HincII (BRL) which cuts the DNA, generating blunt ends.

The reaction was stopped with EDTA at a concentration of 25 mM and the DNA was precipitated, separated and dried.

0.1 $\mu$g of pSM 191 cut by HinCII and 0.09 ug of the 820-bp fragment obtained as described in step A) were suspended in 10 $\mu$l of a buffer solution containing 50 mM pH 7.4 Tris-HCl, 10 mM MgCl$_2$, 10 mM DTT, 1 mM spermidine, 1 mM ATP and 1 U of T4 DNA ligase (Boehringer).

The ligation reaction was conducted at a temperature of 23°C for 14 hours and subsequently stopped at 65°C for 10 minutes. The ligase mixture was then used for transforming E.coli JM 83 cells (pro ara Δ lacpro strA Φ 80d LacZΔM 15) made competent by the method described by Maniatis et al., (Molecular Cloning: a Laboratory Manual 1982).

The recombinants were selected on LB agar plates (DIFCO) containing ampicillin at a concentration of 100 $\mu$g/ml.

Some of the Amp[R] recombinants were able to grow even on plates containg chloramphenicol at a concentration of 20 $\mu$g/ml. These Amp[R] Cm[R] colonies were isolated and the plasmid DNA was extracted by Rodriguez & Tait's method.

From restriction analysis with the enzymes BamHI, PstI and XbaI, it was found that one of these plasmids contained the 820-bp DNA fragment properly inserted and oriented.

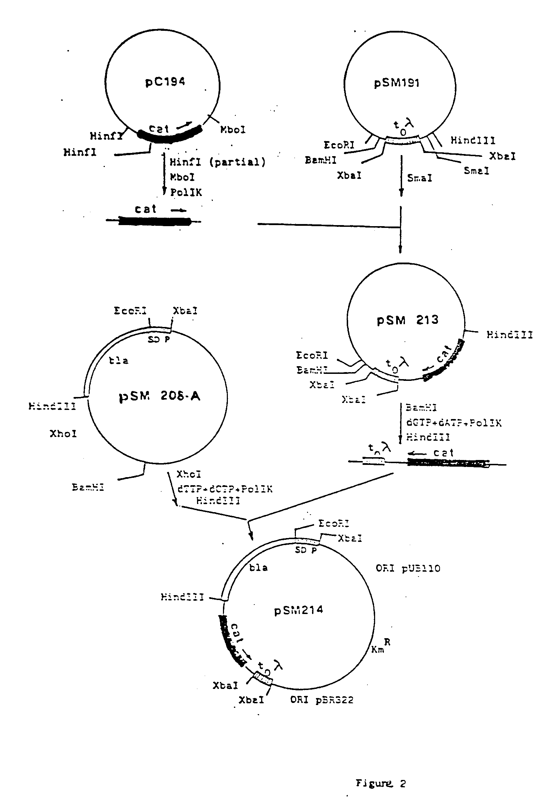

The plasmid thus obtained was designated pSM 213 (Figure 2).

### D) Modification of the promotor of the T5 phage of pSM 208

1γ of the plasmid pSM208 was suspended in 20 $\mu$l of a buffer solution containing 10 mM pH 7.5 Tris-HCl, 7 mM MgCl$_2$, 60 mM NaCl, 1 mM DTT, 2 U of XbaI and 2U of EcoRI. The digestion reaction was carried out at 37°C for 60 minutes and then stopped at 65°C for 10 minutes.

In this way two fragments were obtained, of approximately 7300 bp and 73 bp respectively, the latter corresponding to the promotor region of the T5 phage.

The 7300 bp fragment was purified by electroelution on 0.8% agarose gel.

10 ng of the EcoRI-XbaI 7300 bp fragment and 10 ng of the artificial fragment whose sequence is given below:

```
                          -35                        -10
                CTAGAAAAATTTATTTGCTTTCAGGAAATTTTTTATGTATAATAG
       5'

            3' TTTTTAAATAAACGAAAGTCCTTTAAAAAATACATATTATC
                                    RBS
            ATTCATAAATTTGAGAGCTCAAAGGAGGAATTCTTATG 3'
            TAAGTATTTAAACTCTCGAGTTTCCTCCTTAAGAATACTTAA 5'
                                    EcoRI
```

were suspended in 50 μl of a buffer containing 66 mM Tris-HCl, 10 mM MgCl₂, 10 mM DTT and 2 mM ATP and ligated in the presence of 0.1 U of T₄ DNA ligase at 14°C for 18 hours.

HB101 E.coli cells made competent as described by Maniatis, were transformed with 10 ul of the ligase mixture and the transformants were selected on LB agar plates containing 50 μg/ml of ampicillin.

The plasmid DNA was extracted from an Amp$^R$ colony and sequenced by Maxam and Gilbert's method.

It was thus possible to confirm the exact orientation and insertion of the artificial fragment in the plasmid known as pSM 208-A (Figure 1B).

E) Insertion of the pSM 213 DNA fragment containing the CAT gene and the lambda artificial to terminator into pSM 208-A.

10 μg of the plasmid pSM 213 were digested with 30 U of the enzyme BamHI (Boehringer), in 100 ul of a buffer solution containing 10 mM pH8 Tris-HCl, 100 mM NaCl, 5 mM MgCl₂ and 1 mM β-mercaptoethanol at 37°C for 1 hour.

The reaction was stopped by extraction of the reaction mixture with equal volume of phenol-chloroform and chloroform-isoamyl alcohol and the DNA was precipitated, separated and dried under vacuum. The DNA thus obtained was then re-suspended in 250 μl of a buffer solution containing 50 mM pH 7.2 Tris-HCl, 10 mM MgSO₄, 0.1 mM DTT, 50 μg/ml BSA, 80 μM of dGTP, 80 μM of dATP and 10 U of Klenow DNA polymerase.

The resultant mixture was incubated at ambient temperature for 30 minutes and then stopped with 25 mM EDTA and the proteins extracted with phenol-chloroform and chloroform-isoamyl alcohol.

The DNA was precipitated from the aqueous phase, separated by centrifuging, dried under vacuum and finally re-suspended in 200 μl of a buffer solution containing 50 mM NaCl and 20 U of the HindIII restriction enzyme (Boehringer).

The digestion reaction was carried out at 37°C for 1 hour and then stopped at 65°C for 10 minutes.

The entire reaction mixture was then loaded onto a 6% polyacrylamide gel and run at 120 volts for three hours.

The DNA fragment corresponding to the band of approximately 1000 bp, containing the CAT gene and the to terminator of the lambda phage, was electroeluted as described by Maxam and Gilbert. In parallel, 10 μg of pSM 208-A were digested with 30 U of XhoI (BRL) restriction enzyme in 100 μl of a buffer solution containing 50 mM pH8 Tris-HCl, 10 mM MgCl₂ and 50 mM NaCl at 37°C for 1 hour. After the reaction had been stopped, the DNA was precipitated, separated and then re-suspended in 250 μl of a buffer solution containing 50 mM pH 7.2 Tris-HCl, 10 mM MgSO₄, 0.1 mM DTT, 50 μg/ml BSA, 80 μM each of dTTP and dCTP and 10U of Klenow polymerase.

The reaction was carried out at ambient temperature for 30 minutes, stopped with 25 mM EDTA, treated with phenol-chloroform and chloroform-isoamyl alcohol and, after precipitation, the DNA was re-suspended in 200 μl of a buffer solution containing 50 mM of NaCl, and digested at 37°C for 1 hour with 20 U of HindIII.

After the digestion reaction had been stopped at 65°C for 10 minutes, the mixture was loaded onto 16 ml of a 1 mM NaCl, 30 mM pH8 Tris-HCl, 10 mM EDTA, 5-20% sucrose gradient, and the two DNA fragments separated by centrifuging for 22 hours at a temperature of 20°C in a Beckman ultracentrifuge at 25,000 revolutions per minute (r.p.m.).

At the end of this time, the two DNA fragments of 6300 bp and 212 bp respectively, generated by the previous digestions, were well separated in the gradient.

Of 500 $\mu$l of each fraction collected from the sucrose gradient, 30 ul was analysed by horizontal electrophoresis on a 0.8% agarose gel.

The fractions which showed the presence of the 6300 bp fragment were combined and precipitated with 2.5 volumes of ethanol at -80°C for 15 minutes. After centrifuging in a Sorvall centrifuge model RC-5B for 20 minutes at 12000 rpm, the DNA was dried under vacuum.

2 $\mu$g of the 6300 bp DNA fragment was ligated to 1 $\mu$g of the 1000 bp fragment obtained as described above.

The ligation reaction was carried out in 100 $\mu$l of a buffer solution containing 50 mM pH 7.4 Tris-HCl, 10 mM MgCl$_2$, 10 mM DTT, 1 mM spermidine, 1 mM ATP and 2 U of T4 ligase, at 23°C for 4 hours.

After the reaction had been stopped at 65°C for 10 minutes, the entire ligation mixture was used to transform competent E.coli JM83 cells.

From the recombinants, obtained by selection on LB agar plates containing 20 ug/ml of chloramphenicol and 100 ug/ml of ampicillin, the plasmid DNA was extracted by Rodriguez and Tait's method. One of these plasmids was digested wtih XbaI and HindIII enzymes and analysed by electrophoresis on an agarose gel. This showed the expected characteristics, that is, the presence of triple resistance to the antibiotics kanamycin, chloramphenicol and ampicillin and the transcription of a dicystronic messenger including $\beta$-lactamase and Chloramphenicol-Acetyl-Transferase. The plasmid with approximately 7300 bp was designated pSM 214. (Figure 2).

## Example 2

### Cloning of the DNA sequence which codes for the mature portion of the subunit S2

#### A) Subcloning of the gene for the subunit S2 into the vector pUC12

10 $\mu$g of PT110 (A. Nicosia et al. PNAS USA 1986, 4631-4635) were digested with 10 U of AvaI restriction enzyme in 50 $\mu$l of a solution containing 50 mM pH8 Tris-HCl, 10 mM MgCl$_2$ and 50 mM NaCl at 37°C for 2 hours.

The reaction was stopped by extraction of the mixture with an equal volume of phenol-chloroform and chloroform-isoamyl and the DNA was precipitated from the aqueous phase with 2.5 volumes of ethanol and sodium acetate (final concentration 0.3 M) at -80°C for 30 minutes.

The DNA, after separation by centrifuging, was re-suspended in 50 $\mu$l of a solution containing 60 mM pH 7.6 Tris-HCl, 125 mM NaCl, 6mM MgCl$_2$, 7 mM 2-mercaptoethanol, and 0.01% Triton X-100 and digested at 37°C for 5 minutes with 10 U of SphI restriction enzyme.

After the reaction had been stopped at 65°C for 5 minutes, the mixture was loaded onto a 5% acrylamide gel and run at 120 volts for 3 hours.

The 700 bp fragment, containing the gene for the complete S2 subunit was isolated and eluted by Maxam and Gilbert's method.

In order to facilitate the subcloning of this fragment into the plasmid pUC12, the multiple cloning site (m.c.s.) of pUC12 was replaced by an artificial linker of 22 bp whose sequence is:

```
                        HindIII

        5'AATTCAGCATGCTACCCGGGAA

                GTCGTACGATGGGCCCTTTCGA 3'
        EcoRI
```

In particular, 2.6 ug of pUC12 (2730 bp) (J. Vieria and J. Messing (1982) Gene 19 259 was digested with 3 U each of EcoRI and HindIII, in 40 $\mu$l of a buffer containing 50 mM pH 8 Tris-HCl, 10 mM MgCl$_2$, and 50 mM NaCl at 37°C for 1 hour.

After stopping at 65° for 5 minutes, the mixture was loaded onto a preparative 7.5% acrylamide gel and run at 150 volts for 3 hours.

A fragment of 2680 bp with EcoRI and HindIII ends containing the entire pUC12 vector minus its m.c.s. was thus isolated and eluted.

50 ng of the 2680 bp fragment and 5 ng of the 22 bp artificial linker were suspended in 50 $\mu$l of a buffer solution containing 20 mM pH 7.6 Tris-HCl, 10 mM MgCl$_2$. 10 mM DTT, 0.6 mM ATP and joined, at 20°C for 4 hours in the presence of 2 U of T4 DNA ligase. The entire ligation mixture, after the reaction had been stopped at 65°C for 5 minutes, was used to transform competent E.coli HB101 cells.

The pSM221 plasmid containing the artificial linker with 22 bp was isolated from one of the transformants, selected on LB agar plates with the addition of 100 $\mu$g/ml of Ampicillin. 2.8 $\mu$g of this plasmid were digested with 3 U each of AvaI and SphI, under the digestion conditions described above, to obtain a 2700 bp fragment. 50 ng of this fragment and 500 ng of the 700 bp fragment containing the complete gene for the S2 subunit, were ligated under the ligation conditions described above, at 14°C for 16 hours.

The ligation mixture was then used to transform competent E.coli HB101 cells and the transformants were selected on LB agar plates containing 100 $\mu$g/ml of ampicillin.

Of 16 ampicillin-resistant (Amp$^R$) transformants analysed by rapid extraction of the plasmid DNA, 13 contained the 700 bp fragment.

The 3400 bp plasmid designated pSM222 was extracted and purified from one of these clones. (Figure 3).

## B) Subcloning of the gene for the mature S2 subunit of pSM 214 plasmid.

6 $\mu$g of pSM 222 were digested with 10 U of SstI enzyme in 2 $\mu$l of a buffer 100 mM pH 7.5 Tris-HCl, 50 mM, NaCl, and 10 mM MgCl$_2$ at 37°C for 90 minutes.

After the reaction had been stopped, the DNA was precipitated, separated by centrifuging and re-suspended in 100 $\mu$l of a solution containing 0.2 mM NaCl, 20 mM pH 8 Tris HCl, 12.5 mM CaCl$_2$, 12.5 mM MgCl$_2$ and 1 mM EDTA and incubated for 90 seconds at 23°C with 1.8 U of Bal31 (BRL) nuclease.

In this way, approximately 100 nucleotides which code for the signal sequence responsible for the secretion of S2 into the periplasmic space of Bordetella pertussis were removed from the amino-terminal region of the S2 gene.

The reaction was stopped at 65°C for 5 minutes, the DNA precipitated, separated and then once again re-suspended in 80 $\mu$l of 66 mM pH 8 Tris-HCl, 77 mM NaCl, 5 mM MgCl$_2$ and 10mM DTT buffer and incubated at 37°C for 2 minutes with 2.5 U of ExoIII (BRL).

At the end of the reaction the 5$^{'}$ end of the gene which codes for S2 was exposed by approximately 30 nucleotides.

After the reaction had been stopped with an equal volume of phenol-chloroform and ether, the DNA was precipitated and separated as described above.

The DNA thus obtained was re-suspended in 30 $\mu$l of 50 mM pH8 Tris-HCl, 10 mM MgCl$_2$, 50 mM NaCl buffer and incubated at 37°C for 2 hours with 10 U of HindIII restriction enzyme.

The mixture was stopped at 65°C for 5 minutes, loaded onto 7.5% acrylamide gel and run at 120 volts for 3 hours.

The 650 bp fragment containing the gene for mature S2 was thus isolated.

In order to reconstruct the complete sequence of the mature S2 subunit and enable its subsequent cloning in pSM214, an artificial fragment of 50 nucleotides was used, whose sequence was:

```
    +9 +8 +7 +6 +5 +4 +3 +2 +1              RBS
  5'CGGCGGAATGACGATGCCTGGCGTGGACATAAGAATTCCTCCTTTGAGCT 3'
                                        EcoRI         SstI
```

This single-stranded oligonucleotide is able to pair at one end with the amino-terminal region of mature S2 and thus recreate the complete information for the first 9 amino acids plus methionine for initiating translation, and at the other end with the pSM214 control sequence by means of the SstI end.

3.7 ug of pSM 214 were digested with 5 U of HindIII and 5 U of SstI at 37°C for 120 minutes in 20 ul of 50 mM pH 8, Tris-HCl 10 mM MgCl$_2$ and 50 mM NaCl.

After the reaction had been stopped, the digestion mixture was loaded onto a 0.8% agarose gel and run at 80 volts for 90 minutes to obtain a 6500 bp band.

The DNA fragment corresponding to this band, with SstI-HindIII ends contains the major part of the pSM 214 plasmid minus the translation-regulating sequence RBS and the $\beta$-lactamase gene.

100 ng of the 6500 bp fragment of pSM214, 30 ng of the 650 bp fragment containing the gene for the mature S2 subunit eroded at the 5$^{'}$ end and 20 ng of the 50-base artificial oligonucleotide were ligated in 20 $\mu$l of a buffer solution of 20 mM pH 7.6 Tris-HCl, 10 mM MgCl$_2$, 10 mM DTT, 0.6 mM ATP in the presence of 2 U of T$_4$ DNA ligase at 14 °C for 16 hours.

The ligation mixture was then used for transforming E.coli JM101 cells (lac. pro, SupE, Thi. F'proAB, LacI$^9$, Z$\Delta$M15, traD 36).

The transformants were selected on LB agar plates with the addition of 20 ug/ml of chloramphenicol.

60% of the Cm$^R$ Amp$^S$ clones analysed by rapid extraction of the plasmid DNA contained the gene which codes for the mature S2 subunit under the control of the promotor contained in the Xbal-EcoRI fragment.

The plasmid pSM 226 was isolated from one of these clones. (Figure 3).

This plasmid was used for transforming the B.subtilis SMS118 strain (leu, pyrD1, npr$^-$ spr$^-$) made competent by the method described by Contente and Dubnau (Mol. Gen. Genet. 167, 251-258 (1979)).

All the transformant clones, selected on TBAB (Tryptose Blood Agar Base-Difco) with the addition of 5 $\mu$g/ml chloramphenicol, contained the pSM 226 plasmid.

Example 3

Cloning of the DNA sequence which codes for the mature portion of the S3 subunit

A) Subcloning of the gene for the S3 subunit in pEMBL18

15 $\mu$g of PT110 (A. Nicosia et al. PNAS USA 1983, 4631-4635) were digested with 20 U of BgIII and BamHI respectively in 70 $\mu$l of 50 mM pH 8 Tris-HCl, 10 mM Mg and 50 mM NaCl buffer at 37 °C for 1 hour.

The reaction was stopped at 65 °C for 10 minutes and the digestion mixture was loaded onto a 5% polyacrylamide gel and run at 130 volts for three hours.

The 1738 band containing the gene which codes for S3 was eluted and purified.

Simultaneously, 6 $\mu$g of pEMBL18 plasmid (L.Dente, M. Sallazzo, C. Baldari, G. Cesareni and R. Cortese (1985) "DNA chewing" vol. 1, 101-107) was digested with 10 U of BamHI in 30 ul of a buffer containing 10 mM pH 8 Tris-HCl, 100 mM NaCl, 5 mM MgCl$_2$ and 1 mM 2-mercaptoethanol, at 37 °C for 1 hour. The enzyme reaction was stopped with phenol-chloroform-isoamyl alcohol (25:24:1 V/V) and chloroform-isoamyl alcohol (24:1, V/V) and the DNA was precipitated and isolated. 200ng of pEMBL18, prepared as described above and 300 ng of the 1738 bp fragment were ligated, in 50 $\mu$l of a buffer containing 20 mM pH 7.6 Tris-HCl, 10 mM MgCl$_2$, 10 mM DTT, 1 mM ATP, with 3 U of T4 ligase at 14 °C for 18 hours.

After the reaction had been stopped at 65 °C for 10 minutes, the ligation mixture was used for transforming competent E.coli JM 101 cells.

From the recombinant clones obtained by selection on LB agar plates (Difco) containing 100 $\mu$g/ml of ampicillin, 40 ul/ml of 5-bromo-4-chloro-3-indolyl b-D-galactoside (X-Gal), and 125 $\mu$g/ml of isopropyl b-D-galactoside (IPTG) (Viera J. and Messing J, Gene 19, p. 259 (1982)), 24 white Amp$^R$ colonies were isolated.

These colonies were examined by rapid extraction of the plasmid DNA by the method described by Maniatis et al. (Molecular Cloning: a Laboratory Manual, Cold Spring Harbor 1982) and subsequent digestion of the plasmids with PstI restriction enzyme.

5 of the 24 plasmids analysed showed two fragments, of 4995 and 800 bp respectively, which displayed the exact orientation of the fragment containing the gene of the S3 subunit.

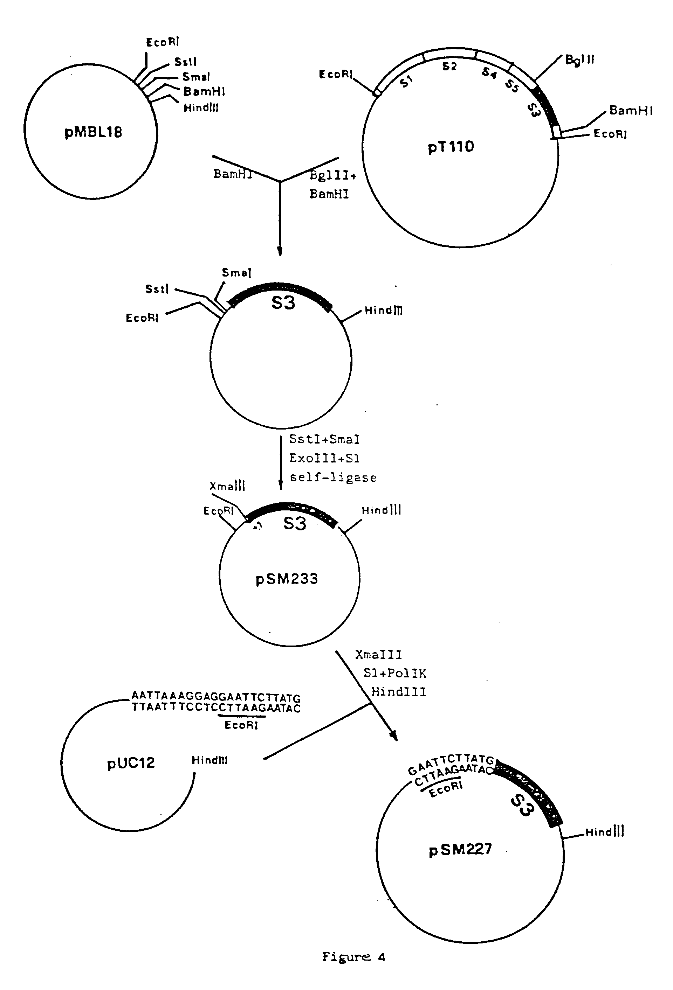

The plasmid designated pSM 231 (Figure 4) was extracted from one of these 5 clones by the method described by Maniatis et al.

3 $\mu$g of the pSM 231 plasmid was digested with 10 U of SstI (BRL) restriction enzyme in 30 $\mu$l of a buffer containing 14 mM pH 7.5 Tris-HCl, 6mM MgCl$_2$, 6 mM 2-mercaptoethanol and 30 mM NaCl at 37 °C for 1 hour.

After the reaction had been stopped at 65 °C for 10 minutes, the plasmid DNA was precipitated and separated as described above and then digested in 30 $\mu$l of a buffer solution containing 15 mM pH 8.5 Tris-HCl, 15 mM KCl, 6 mM MgCl$_2$ and 6 mM 2-marcaptoethanol with 10 U of the SmaI restriction enzyme at 25 °C for 1 hour.

3 $\mu$g of pSM 231 precipitated after digestion SmaI was resuspended in 40 $\mu$l of a buffer containing 66 mM pH8 Tris-HCl, 77 mM NaCl, 5 mM MgCl$_2$, 10 mM DTT and 15 U of Exonuclease III.

The digestion reaction was carried out at 37 °C with samples of 4 $\mu$l, 4.8 $\mu$l, 7.2 $\mu$l, 10 $\mu$l, and 14 $\mu$l being taken at different times. These samples, combined, were added to 4.5 $\mu$l of a buffer containing 0.5 pH 4 sodium acetate, 0.5 M NaCl, 60 mM ZnSO$_4$ and 60 U of S1 nuclease. The resultant mixture was incubated at 23 °C for 15 minutes and then stopped by the addition of 10 $\mu$l of a solution containing 0.1 M EDTA and 1.5 M sodium acetate.

The mixture was extracted with phenol-chloroform-isoamyl alcohol (25: 24: 1, V/V) and chloroform-isoamyl alcohol (24:1 V/V) and the plasmid DNA, after precipitation and separation, was re-suspended in 25 µl of a buffer containing 50 mM pH 7.2 Tris-HCl, 10 mM MgSO₄, 0.1 mM DTT, 50 µg/ml of BSA, 0.25 mM of dATP, dGTP and DTTP respectively and 3 U of DNA polymerase I enzyme (large fragment) (NEN).

The mixture was incubated at ambient temperature (20°-25°C) for 30 minutes and stopped by the addition of 1 ul of 0.5 M EDTA. 100 ng of plasmid DNA were linked to itself in 50 ul of buffer containing 20 mM pH 7.6 Tris-HCl, 10 mM MgCl₂, 10 mM DTT, 1 mM ATP, in the presence of 3 U of T4 DNA ligase, at 23°C for 18 hours.

The entire ligation mixture was then used for transforming competent E.coli JM 101 cells.

500 recombinant clones, obtained by selection on LB agar plates containing 100 µg/ml of ampicillin, 40 µg/ml X-Gal, 125 µg/ml of IPTG, were replicated on a BA 85 nitrocellulose filter (Schellincher and Schnell).

The replicate colonies were then lysed by treating the filters with 0.5 N NaOH for 5 minutes, 1.5 M pH 7.5 Tris-HCl for 5 minutes, 0.3M SSC 2X NaCl, pH 7, 30 mM Na citrate for 5 minutes and 70% ethanol for 5 minutes and the filters were dried at 60°C under vacuum for 2 hours.

The production of the S3 subunit by these clones was then assayed by immunological testing.

In particular, the filters were incubated in the presence of rabbit anti-S3 antibodies and goat antirabbit IgG antibodies combined with peroxidase. By this procedure, 15 clones were found which produced the S3 subunit of pertussis toxin.

The plasmid DNA extracted from 4 of these clones was sequenced by the dideoxynucleotide method with the use of the denatured plasmids as a template (Masahiro Hattori and Yoshiyuki Sakaki (1986) Anal. Biochem. 152, 232-238.

The sequence of one of these plasmids, designated pSM 233, was as follows:

$\beta$ – galactoxydase                    -1    +1

| Met | Thr | Met | ILe | Thr | Asn | Ser | Ala | Val | Ala |
|-----|-----|-----|-----|-----|-----|-----|-----|-----|-----|
| ATG | ACC | ATG | ATT | ACG | AAT | TCG | GCC | GTT | GCC |

XmaIII

It was thus confirmed that, in the pSM 233 recombinant plasmid, the non-coding regions and the sequence coding for the peptide secretion signal less 1 amino acid were eliminated as a result of the treatment with Exonuclease III and nuclease S1, thus obtaining the S3 subunit fused to 7 aminoacids of the aminoterminal portion of $\beta$-galactoxidase.

Furthermore, a restriction site recognised by XmaIII enzyme was created in the recombinant plasmid pSM 233 in the region exactly corresponding to the beginning of the mature S3 subunit.

B) Subcloning of the gene for the mature S3 subunit into pSM 214.

The gene which codes for the mature S3 subunit of pertussis was isolated from the pSM 233 recombinant plasmid by digestion with XmaIII and PstI restriction enzymes which cut in positions corresponding to the +1 amino acid and 53 amino acids downstream of the stop codon respectively.

15 µg of pSM 233 were digested with 20 U of XmaIII (Biolabs) in 250 µl of a solution containing 10 mM pH 8.2 Tris-HCl, 8 mM MgCl₂, 6 mM 2-mercaptoethanol and 100 ug/ml BSA at 37°C for 4 hours.

After the reaction had been stopped at 65°C for 5 minutes, the DNA was precipitated, separated and loaded on to a 7.5% acrylamide gel.

After electrophoresis at 120 volts for three hours, a 942 bp band containing the region coding for mature S3 and the non-coding region downstream of S3 (330 bp) was isolated and eluted by the method described by Maxam and Gilbert.

Subsequently, 1 µg of the fragment corresponding to the 942 bp band was digested in 20 ul of 50 mM pH4 sodium acetate, 50 mM of NaCl and 6 mM of ZnSO₄, with 20 U of S1 nuclease at 23°C for 15 minutes.

The reaction was stopped by addition of EDTA 20 mM final and, after precipitation, the DNA was re-suspended in 10 µl of 50 mM pH 7.2 Tris-HCl, 10 mM MgSO₄, 0.1 mM DTT, 50 µg/ml BSA and 0.2 mM didNTPs buffer and treated with 4U of Klenow DNA polymerase at 23°C for 30 minutes.

500 ng of the fragment thus obtained were digested with 1 U of PstI at 37°C for 1 hour and the 800 bp fragment, which codes for the mature S3 subunit, was electroeluted as described above. Simultaneously, 2.5 ug of the vector pUC12 was digested with 4 U of EcoRI and PstI restriction enzymes in 30 $\mu$l of 50 mM pH8 Tris-HCl, 10 mM MgCl$_2$, 50 mM NaCl at 37°C for 1 hour.

The reaction was stopped at 65°C for 5 minutes and the DNA was precipitated, first with 2.5 volumes of ethanol in 0.3 M sodium acetate at -80°C for 30 minutes, then with 0.5 volumes of isopropanol in NaClO$_4$ 0 5 M final at ambient temperature for 15 minutes.

The DNA thus obtained, after separation by centrifuging, was re-suspended in 125 $\mu$l of a ligase buffer containing 10 U of T4 DNA ligase and 370 ng of the artificial linker given below:

<center>38</center>

<center>RBS    EcoRI</center>

<center>AATTAAAGGAGGAATTCTTATG</center>

<center>TTTCCTCCTTAAGAATAC</center>

This linker can pair with the EcoRI end of the pUC12 plasmid creating a blunt end preceded by the translation initiation triplet (ATG). The ligase mixture was incubated at 14°C for 16 hours and then stopped at 65°C for 5 minutes. The 5$'$OH free end of the linker was then phosphorylated with 5 U of T4 polynucleotide Kinase at 37°C for 1 hour. After extraction with phenol-chloroform, the DNA was precipitated with isopropanol and digested with 4 units of PstI in 30 $\mu$l of 50 mM pH8 Tris-HCl, 10 mM MgCl$_2$, and 50 mM NaCl at 37°C for 1 hour.

The reaction was stopped by extraction with phenol-chloroform and the DNA was precipitated with ethanol in 0.3 M sodium acetate. 150 ng of the plasmid DNA thus prepared and 60 ng of the fragment containing the gene for the mature S3 subunit were ligated, in the presence of T4 DNA ligase, at 23°C for 16 hours.

The ligation mixture was used for transforming competent E.coli HB 101 cells.

The pSM 227 plasmid was isolated from one of the recombinant Amp$^R$ clones obtained by selection on LB agar plates with the addition of 100 $\mu$l/ml of Ampicillin. In this, the mature S3 gene, preceded by the amino acid alanine which was not removed by treatment with SI nuclease and by methionine in the aminoterminal position, is flanked by EcoRI and HindIII restriction sites upstream and downstream respectively; (Figure 4).

3 $\mu$g of pSM227 were digested with 3U of EcoRI and 3 U of HindIII in 30 ul of a buffer containing 50 mM pH 8.0 Tris-HCl, 10 mM mgCl$_2$, 50 mM NaC2 at 37°C for 1 hour.

The mixture was stopped at 65°C for 5 minutes and then loaded on to a 7.5% acrylamide gel.

After electrophoresis at 120 volts for three hours, a 65 bp band which contained the gene for the mature S3 subunit was separated and eluted. 100 ng of the fragment corresponding to this band was then ligated to 300 ng of pSM 214 plasmid, previously digested with EcoRI and HindIII enzymes under the conditions described above.

The ligation reaction was carried out in the presence of 2U of T4 DNA ligase at 4°C for 16 hours.

2 and 4 $\mu$l portions of the mixture were used for transforming competent E.coli JM 101 cells.

Selection of the recombinants was effected on LB agar plates containing 20 $\mu$g/ml of Chloramphenicol.

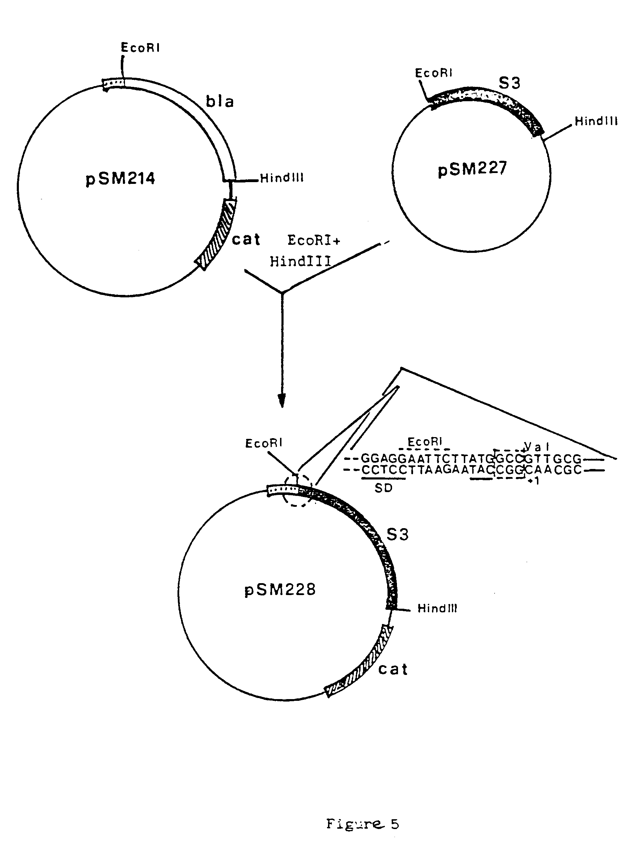

The plasmid pSM 228 containing the gene for the mature S3 subunit plus alanine in position -1 under the control of the 74-nucleotide artificial promotor (Figure 5) was isolated from one of the Cm$^R$ clones.

100 ng of the pSM 228 plasmid were used for transforming competent B.subtilis SMS 118 cells.

All the recombinant clones, analysed on TBAB with the addition of chloramphenicol (5$\mu$g/ml), contained the pSM 228 plasmid (Figure 5).

EXAMPLE 4

Cloning of the DNA sequence which codes for the mature portion of the S4 subunit

A) Subcloning of the gene for the S4 subunit in pEMBL19.

10 $\mu$g of PT110 was digested with 12 U of AvaI enzyme and 18 U of PstI enzyme in 100 ul of a buffer containing 50 mM pH8.0 Tris-HCl, 10 mM MgCl$_2$, 50 mM NaCl at 37°C for 1 hour.

The reaction was stopped at 65°C for 5 minutes and the mixture was loaded onto a 6% polyacrylamide gel and run at 120 volts for 2 hours.

<center>15</center>

A 765 bp fragment of DNA containing the gene for the S4 subunit was electroeluted as described by Maxam and Gilbert.

Simultaneously 1 $\mu$g of pEMBL19 was digested with AvaI and PstI restriction enzymes under the conditions described above.

The reaction was stopped at 65°C for 10 minutes.

300 ng of plasmid DNA thus prepared and 180 ng of the 765 bp fragment containing the gene for S4 were ligated in the presence of 1 U of T4 DNA ligase in 30 $\mu$l of a buffer containing 0.66 mM pH 7.6 Tris-HCl, 50 mM MgCl$_2$, 50 mM DTT and 1 mM ATP at 14°C for 8 hours.

After the reaction had been stopped at 65°C for 10 minutes, the ligation mixture was used for transforming competent E.coli JM 101 cells.

The plasmid DNA was extracted from the transformants, obtained by selection on LB agar plates containing 40 $\mu$g/ml X-gal, 125$\mu$g/ml IPTG and 100 $\mu$g/ml Ampicillin.

One of these plasmids, designated pSM244-A, contained the 765 bp fragment correctly inserted and oriented.

3 $\mu$g of pSM 244-A was digested in 30 ul of a solution containing 50 mM pH 8 Tris-HCl, 10 mM MgCl$_2$, and 50 mM NaCl, with 5 U of EcoRI at 37°C for 1 hour and subsequently with 9 U of BstEII at 60°C for 1 hour.

The digestion generated two fragments of 3860 bp and 140 bp respectively, the latter containing part of the signal sequence for the secretion of S4 in Bordetella pertussis and the first 36 amino acids of the gene for mature S4.

1 $\mu$g of the 3860 bp fragment was ligated in 100 $\mu$l of 70 mM pH 7.6 Tris-HCl, 10 mM MgCl$_2$, 5 mM DTT, and 1 mM ATP, in the presence of 10 U of T$_4$ DNA ligase with 100 ng of an artificial fragment of 47 bp which reforms the coding sequence for the aminoterminal portion of the mature S4 subunit and whose sequence is:

```
  +1   2   3   4   5   6   7   8   9   10  11    12
  AATTCTTATGGACGTCCTTATGTGCTGGTGAAGACCAATATGGTG
          GAATACCTGCAAGGAATACACGACCACTTCTGGTTATACCACCAGTG
```

The ligation reaction was carried out at 14°C for 8 hours and then stopped at 65°C for 5 minutes.

The ligation mixture was then used to transform competent E.coli JM 101 cells and the transformants were selected on LB agar plates containing 100 ug/ml of ampicillin.

Analysis of the plasmid DNA, extracted from some Amp[R] clones confirmed insertion of the 47 bp artificial fragment in the position of the original 140 bp fragment.

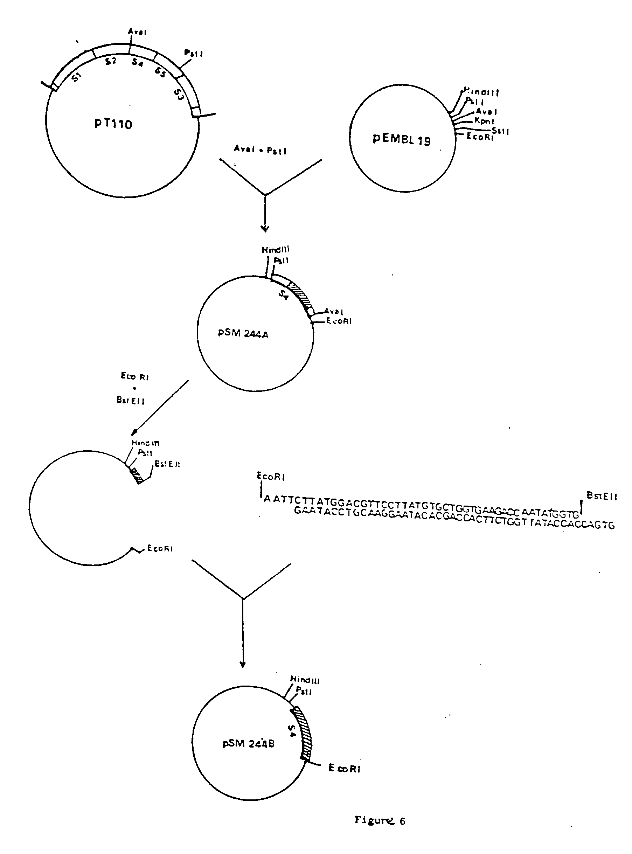

The recombinant plasmid extracted from one of these clones was designated pSM 244-B (Figure 6).

B) Subcloning of the gene for mature S4 in pSM 214

2 $\mu$g of pSM 244-B were suspended in 30 $\mu$l of a solution containing 50 mM pH 8.2 Tris-HCl, 8 mM MgCl$_2$, 6 mM 2-mercaptoethanol and 100 $\mu$g/ml of BSA and digested at 37°C for 1 hour with 5 U of EcoRI and 5 U of HindIII which cut upstream of the initiating codon and approximately 340 bases downstream of the termination codon of the gene which codes for mature S4 respectively.

After the reaction had been stopped at 65°C for 5 minutes, the DNA was precipitated, re-suspended and loaded on to a 7.5% acrylamide gel.

After electrophoresis at 120 volts for 3 hours, a 687 bp fragment containing the region which codes for mature S4 was isolated and eluted.

120 ng of the 687 bp fragment was ligated with 500 ng of pSM 214 previously digested as described above, in a ligation mixture in the presence of 1 U of T4 DNA ligase at 14°C for 8 hours. The ligation mixture was used to transform E.coli HB 101 cells made competent by the Ca chloride method. Selection was carried out on LB agar plates containing 20 $\mu$g/ml Cm.

Analysis of the plasmid DNA, extracted from the Cm[R] Amp[S] positive clones confirmed the correct insertion of the gene for the mature S4 subunit.

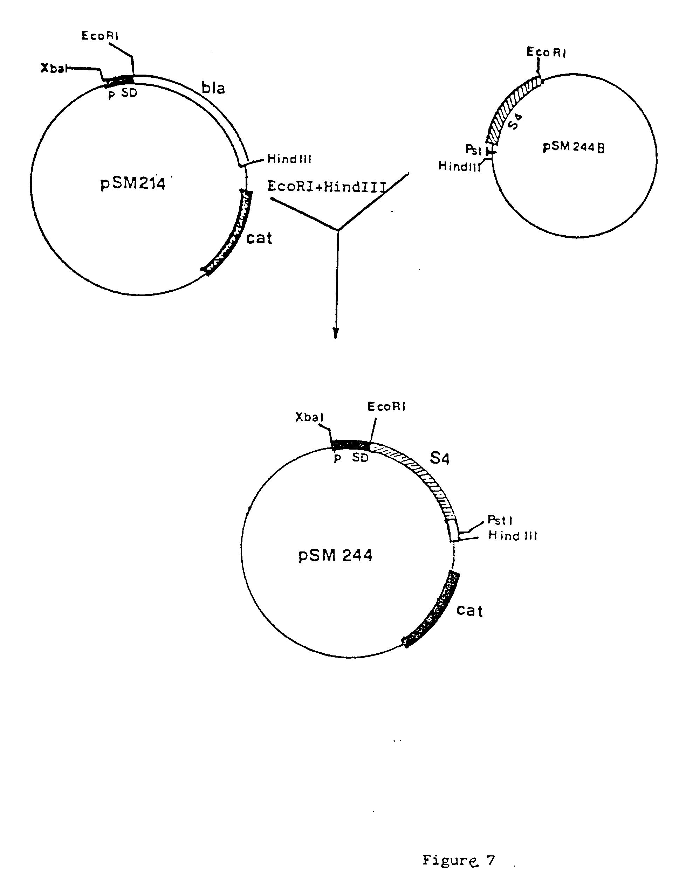

The plasmid extracted from one of these clones, pSM 244, was used for transforming competent B.subtilis SMS 118 cells. The positive transformants, selected on TBAB plates with the addition of 5 $\mu$g/ml or chloramphenicol, contained pSM 244 plasmid (Figure 7).

EXAMPLE 5

Cloning of the DNA sequence which codes for the mature portion of the S5 subunit

A) Subcloning of the gene for the S5 subunit in pUC12

Construction of the pSM 249 plasmid

10 $\mu$g of the pUB110 plasmid (T. Tanaka and N. Sucaka, Journal of Bacteriology, 1983, Vol. 154, 1184-1194) and 3.7 $\mu$g of PT110 were each digested in 30 $\mu$l of a solution containing 6 mM pH8 Tris-HCl, 150 mM NaCl, 6 mM MgCl$_2$, and 7 mM $\beta$-mercaptoethanol with 5 units of XbaI and BamHI restriction enzymes at 37°C for 1 hour.

The reaction was stopped by extraction with 1 volume of phenol-chloroform-isoamyl alcohol and, after precipitation, the DNA was re-suspended in 15 $\mu$l of TE (10 mM pH 8 Tris-HCl, and 1 mM EDTA) buffer. Subsequently, 13 ul of each sample thus digested was ligated in 30 $\mu$l of ligation mixture with 2 units of T4 DNA ligase at 4°C for 16 hours.

This ligation mixture was then used for transforming competent B.subtilis SM3 108 (his, leu, met, npr⁻, recE4) cells.

The transformants were selected on TBAB plates containing kanamycin at 5 $\mu$g/ml.

The plasmid pSM249 containing an insert of 3500 bp having XbaI and BamHI ends which comprises a large part of the pertussis function, including genes for the S5 subunit, was extracted and purified from one of these clones.

10 $\mu$g of pSM 249 were digested in 100 ul of a solution containing 10 mM pH 8.0 Tris-HCl, 20 mM NaCl, 10 mM MgCl$_2$, 5 mM $\beta$-mercaptoethanol and 100 $\mu$g/ml BSA, with 50 U of NaeI restriction enzyme at 37°C for 1 hour.

The reaction was stopped at 65°C for 5 minutes and, after precipitation, the DNA was digested with 9 U of the BclI enzyme in 10 $\mu$l of a solution containing 50 mM pH 8 Tris-HCl, 10 mM MgCl$_2$, and 50 mM NaCl at 37°C for 30 minutes.

The digestion mixture was stopped at 65°C for 5 minutes and loaded onto a 7.5% polyacrylamide gel.

After electrophoresis at 120 volts for 3 hours, a fragment with approximately 350 bp was eluted containing the gene for S5 starting from amino acid 9.

2.5 $\mu$g of pUC12 was digested with 3 U of EcoRI and BamHI restriction enzymes in 30 $\mu$l of 50 mM pH 8 Tris-HCl, 10 mM MgCl$_2$, and 50 mM NaCl at 37°C for 1 hour and a 2680 bp fragment was electroeluted as described above.

20ng of the 2680 bp fragment, 10 ng of the 350 bp fragment containing S5 and 3 ng of the 34 bp artificial oligonucleotide whose sequence is:

```
EcoRI        +1

AATTCTTATGATATACAGCCCGGCTGACGTCGCC

GAATACTATATGTCGGGCCGACTGCAGCGG
```

were then ligated in 20 $\mu$l of 20 mM pH 7.6 Tris-HCl, 10 mM MgCl$_2$, 10 mM DTT, 0.6 mM ATP and 2 U of T$_4$ DNA ligase at 14°C for 16 hours. The ligation mixture was used for transforming competent HB 101 E.coli cells and the transformants were selected on LB agar plates containing 100 $\mu$g/ml of ampicillin.

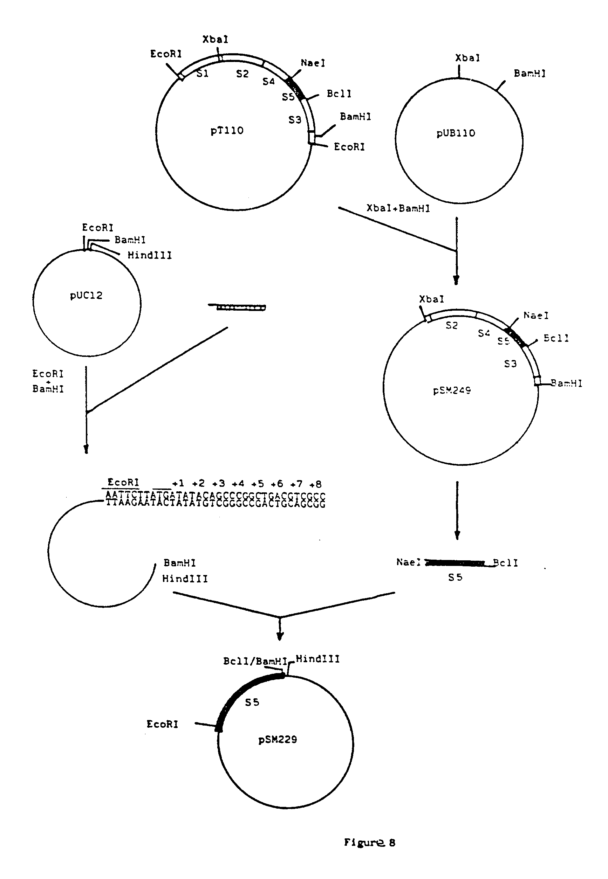

The plasmid pSM 229 (Figure 8) which contains the gene which codes for the mature S5 subunit, equivalent to the natural one except for the initiating methionine flanked by the EcoRI and HindIII restriction sites, was isolated from one of the Amp[R] clones.

B) Subcloning of the gene for the mature S5 subunit in pSM 214

5 $\mu$g of pSM 229 were digested with 10 U of EcoRI and HindIII restriction enzymes in 50 $\mu$l of a solution of 50 mM pH 8 Tris-HCl, 50 mM NaCl, and 10 mM MgCl$_2$ at 37°C for 30 minutes. The mixture, after being stopped at 65°C for 5 minutes was loaded onto a 7.5% polyacrylamide gel and run at 120 volts for 3 hours.

A 400 bp band containing the gene for mature S5 was electroeluted as described by Maxam and Gilbert.

17

200 ng of the fragment corresponding to this band and 600 ng of pSM 214, digested with EcoRI and HindIII under the same conditions as described above, were ligated in the presence of 5 U of T$_4$ DNA ligase in 6 $\mu$l of ligation solution at 14°C for 16 hours.

After the reaction has been stopped at 65°C for 5 minutes, the mixture was used to transform competent E.coli HB 101 cells.

Selection of the transformants was carried out on L agar plates containing 20 $\mu$g/ml of chloramphenicol.

The plasmid pSM 243 containing the gene for the mature S5 subunit was isolated from one of the Cm$^R$ clones.

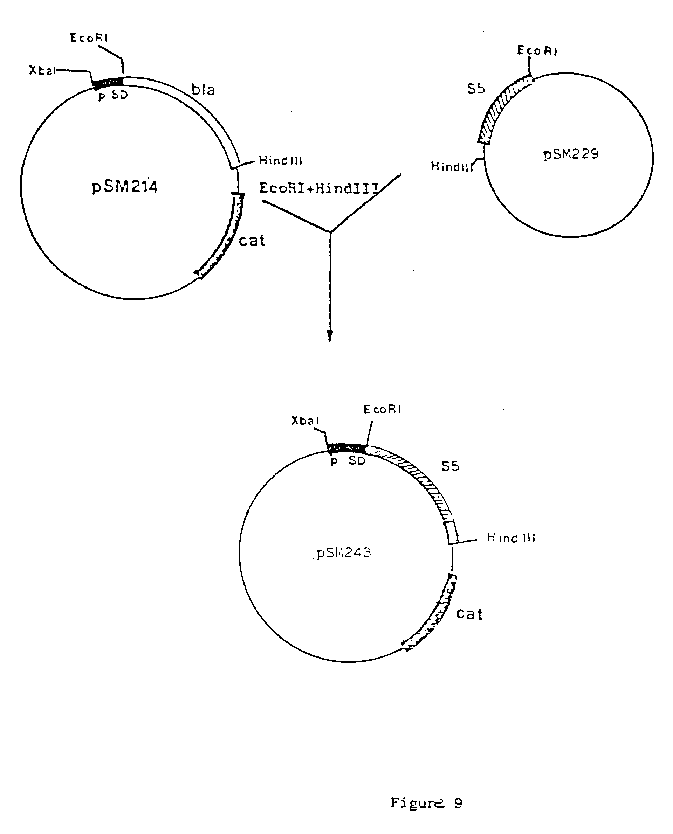

100 ng of pSM 243 were then used to transform competent B.subtilis SMS 118 cells. All the recombinant clones, selected on TBAB containing 5 $\mu$g/ml of chloramphenicol, contained the pSM 243 plasmid (Figure 9).

EXAMPLE 6

Expression of the mature S2, S3, S4, and S5 subunits in B.subtilis SMS 118.

B.subtilis SMS 118 cells containing the plasmids pSM226, pSM228, pSM244 and pSM243 respectively were grown in 10 ml of VY medium (Veal infusion broth (DIFCO) 25 g/l, yeast extract (DIFCO) 5 g/l) with the addition of 5 $\mu$g/ml of chloramphenicol at 37°C for 16 hours.

1 ml of each culture was then centrifuged in an Eppendorf centrifuge at 4°C for 1 minute at maximum speed. The cells thus obtained were washed in 30 mM pH 8.0 Tris-HCl and 50 mM NaCl and re-suspended in 100 ul of SET buffer (20% sucrose, 50 mM pH 7.6 Tris-HCl, 50 mM EDTA) with the addition, in the case of S2 and S3 of 20 mg/ml of lysozyme. The cellular suspensions thus obtained were kept at 37°C for 5 minutes under agitation.

To 24 $\mu$l of each lysate were then added 36 $\mu$l of SDS buffer (125mM pH6.8 Tris-HCl, 3% sodium dodecyl sulphate, 3% 2-mercaptoethanol, 20% glycerol) and 2 $\mu$l of bromophenol blue, and the resultant solutions were left at 100°C for 5 minutes.

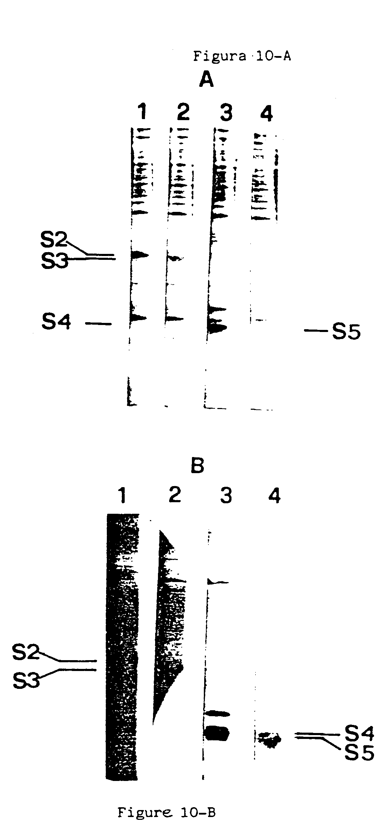

20 $\mu$l of each lysate were then loaded on to a 15% polyacrylamide sodium dodecyl sulphate (SDS) gel (Laemli, Nature, (1970), 277, 680) and, after an electrophoretic flow at 25 mA for 3 hours, the proteins were displayed both by dyeing with Coomassie Blue ("Gel electrophoresis of proteins: a practical approach" edited by B.D. Hames and D. Rickwod, published by IRL Press Limited) and by transfer onto Schleicher and Schull nitrocellulose 45 $\mu$m filters and subsequent treatment of the filters with rabbit anti-S2, anti-S3, anti-S4 and anti-S5 antibodies respectively and with goat anti-rabbit IgG antibodies combined with peroxidase (Miles) as described by Towbin (H. Towbin, T. Stacheling and J. Gordon (1979) PNAS USA VOL. 76, 4350-4). On dyeing with Coomassie Blue, proteins with molecular weights of 23,000, 22,000, 12,000 and 10,000 daltons respectively appear which co-migrate with the mature subunits S2, S3, S4 and S5 of the natural toxin (Figure 10A).

These proteins react specifically with anti-S2, -S3, -S4 and -S5 antibodies, as demonstrated by immunoelectrophoretic analysis and shown in Figure 10-B.

The quantity of protein synthesised varies from a maximum of 80 mg/1 (S2 and S4) to a minimum of 20-40 mg/l (S3 and S5) under the culture conditions described above.

**Claims**

1.  A vector useful for the cloning of a DNA sequence which codes for a heterologous protein, characterised in that it contains the origins for functional replication in Bacillus and in Escherichia coli, the genes which code for resistance to the antibiotics kanamycin, ampicillin and chloramphenicol, and a strong artificial promotor which directs the transcription of a dicystronic messenger RNA which codes for resistance to ampicillin and chloramphenicol.

2.  A vector according to Claim 1, in which the origin for functional replication in Bacillus is that of plasmid pUB110.

3.  A vector according to Claim 1, in which the origin for functional replication in Escherichia coli is that of plasmid pBR322.

4. A vector according to Claim 1, in which the nucleotide sequence of the artificial promotor is:

**ATTCATAAATTTGAGAGCTCAAAGGAGGAATTCTTATG 3'**

**TAAGTATTTAAACTCTCGAGTTTCCTCCTTAAGAATACTTAA 5'**

5. A vector according to Claim 1, in which the DNA sequence codes for an immunologically or biologically active protein.

6. A vector according to Claim 5, in which the heterologous protein is a mature subunit of pertussis toxin.

7. A vector according to Claim 1, deposited as Bacillus subtilis SMS 118 (pSM 214) (ATCC 67320).

8. A recombinant plasmid useful for the expression of a heterologous protein, obtained by the removal from the cloning vector, as defined in Claims 1 to 7, of the $\beta$-lactamase gene, with EcoRI and HindIII restriction enzymes, and the cloning of DNA sequences which code for the heterologous protein in the restriction sites.

9. A recombinant plasmid according to Claim 8, in which the heterologous protein is a subunit of pertussis toxin.

10. Recombinant plasmid pSM226 (ATCC 67321) according to Claim 9, in which the DNA sequence codes for the S2 mature subunit.

11. Recombinant plasmid pSM228 (ATCC 67322) according to Claim 9, in which the DNA sequence codes for the S3 mature subunit.

12. Recombinant plasmid pSM244 (ATCC 67324) according to Claim 9, in which the DNA sequence codes for the S4 mature subunit.

13. Recombinant plasmid pSM243 (ATCC 67323) according to Claim 9, in which the DNA sequence codes for the S5 mature subunit.

14. A micro-organism transformed with a recombinant plasmid as defined in Claims 8 to 13.

15. A micro-organism according to Claim 14, selected from the group of Escherichia coli and Bacillus subtilis.

16. A micro-organism according to Claim 15, being Bacillus subtilis SMS 118.

17. Bacillus subtilis SMS 118 (pSM226) (ATCC 67321).

18. Bacillus subtilis SMS 118 (pSM228) (ATCC 67322).

19. Bacillus subtilis SMS 118 (pSM244) (ATCC 67324).

20. Bacillus subtilis SMS 118 (pSM243) (ATCC 67323).

21. A method for the preparation of heterologous proteins characterised in that a micro-organism transformed as defined in Claims 14 to 20 is grown in a liquid medium containing sources of nitrogen, carbon and mineral salts in the presence of chloramphenicol.

22. A method according to Claim 21, in which the microorganism is B. subtilis SMS 118 (pSM226) (ATCC 67321) and the heterologous protein is the mature S2 subunit.

**23.** A method according to Claim 21, in which the microorganism is B. subtilis SMS 118 (pSM228) (ATCC 67322) and the heterologous protein is the mature S3 subunit.

**24.** A method according to Claim 21, in which the microorganism is B. subtilis SMS 118 (pSM244) (ATCC 67324) and the heterologous protein is the mature S4 subunit.

**25.** A method according to Claim 21, in which the microorganism is B. subtilis SMS 118 (pSM243) (ATCC 67323) and the heterologous protein is the mature S5 subunit.

**Patentansprüche**

**1.** Vektor, der für die Clonierung einer ein heterologes Protein codierenden DNA-Sequenz nützlich ist, dadurch gekennzeichnet, daß er die Ursprünge für die funktionelle Replikation in Bacillus und in Escherichia coli, die Gene, die die Resistenz gegenüber den Antibiotika Kanamycin, Ampicillin und Chloramphenicol codieren, und einen starken künstlichen Promotor enthält, der die Transkription einer dicistronischen Messenger-RNA kontrolliert, die die Resistenz gegenüber Ampicillin und Chloramphenicol codiert.

**2.** Vektor nach Anspruch 1, in dem der Ursprung für die funktionelle Replikation in Bacillus der vom Plasmid pUB110 ist.

**3.** Vektor nach Anspruch 1, in dem der Ursprung für die funktionelle Replikation in Escherichia coli der vom Plasmid pBR322 ist.

**4.** Vektor nach Anspruch 1, in dem die Nucleotidsequenz des künstlichen Promotors die folgende ist:

```
ATTCATAAATTTGAGAGCTCAAAGGAGGAATTCTTATG 3'

TAAGTATTTAAACTCTCGAGTTTCCTCCTTAAGAATACTTAA 5'
```

**5.** Vektor nach Anspruch 1, in dem die DNA-Sequenz ein immunologisch oder biologisch aktives Protein codiert.

**6.** Vektor nach Anspruch 5, in dem das heterologe Protein eine reife Untereinheit des Pertussistoxins ist.

**7.** Vektor nach Anspruch 1, hinterlegt als Bacillus subtilis SMS 118 (pSM 214) (ATCC 67320).

**8.** Rekombinantes Plasmid, das für die Expression eines heterologen Proteins nützlich ist, erhalten durch die Entfernung des $\beta$-Lactamasegens mit EcoRI- und HindIII-Restriktionsenzymen aus dem Clonierungsvektor gemäß der Definition in den Ansprüchen 1 bis 7, und die Clonierung von DNA-Sequenzen, die das heterologe Protein codieren, in die Restriktionsstellen.

**9.** Rekombinantes Plasmid nach Anspruch 8, wobei das heterologe Protein eine Untereinheit von Pertussistoxin ist.

**10.** Rekombinantes Plasmid pSM226 (ATCC 67321) nach Anspruch 9, in dem die DNA-Sequenz die reife S2-Untereinheit codiert.

**11.** Rekombinantes Plasmid pSM228 (ATCC 67322) nach Anspruch 9, in dem die DNA-Sequenz die reife S3-Untereinheit codiert.

**12.** Rekombinantes Plasmid pSM244 (ATCC 67324) nach Anspruch 9, in dem die DNA-Sequenz die reife S4-Untereinheit codiert.

**13.** Rekombinantes Plasmid pSM243 (ATCC 67323) nach Anspruch 9, in dem die DNA-Sequenz die reife S5-Untereinheit codiert.

**14.** Mikroorganismus, der mit einem rekombinanten Plasmid nach den Ansprüchen 8 bis 13 transformiert ist.

**15.** Mikroorganismus nach Anspruch 14, ausgewählt aus Escherichia coli und Bacillus subtilis.

**16.** Mikroorganismus nach Anspruch 15, nämlich Bacillus subtilis SMS 118.

**17.** Bacillus subtilis SMS 118 (pSM226) (ATCC 67321).

**18.** Bacillus subtilis SMS 118 (pSM228) (ATCC 67322).

**19.** Bacillus subtilis SMS 118 (pSM244)(ATCC 67324).

**20.** Bacillus subtilis SMS 118 (pSM243) (ATCC 67323).

**21.** Verfahren zur Herstellung von heterologen Proteinen, dadurch gekennzeichnet, daß ein gemäß den Ansprüchen 14 bis 20 transformierter Mikroorganismus in einem Flüssigmedium, das Stickstoff-, Kohlenstoffquellen und Mineralsalze enthält, in Gegenwart von Chloramphenicol gezüchtet wird.

**22.** Verfahren nach Anspruch 21, in dem der Mikroorganismus B. subtilis SMS 118 (pSM226) (ATCC 67321) ist und das heterologe Protein die reife S2-Untereinheit ist.

**23.** Verfahren nach Anspruch 21, in dem der Mikroorganismus B. subtilis SMS 118 (pSM228) (ATCC 67322) ist und das heterologe Protein die reife S3-Untereinheit ist.

**24.** Verfahren nach Anspruch 21, in dem der Mikroorganismus B. subtilis SMS 118 (pSM244) (ATCC 67324) ist und das heterologe Protein die reife S4-Untereinheit ist.

**25.** Verfahren nach Anspruch 21, in dem der Mikroorganismus B. subtilis SMS 118 (pSM243) (ATCC 67323) ist und das heterologe Protein die reife S5-Untereinheit ist.

**Revendications**

**1.** Vecteur utile pour le clonage d'une séquence d'ADN codant pour une protéine hétérologue, caractérisé en ce qu'il contient les origines de réplication fonctionnelle que l'on trouve dans Bacillus et dans Escherichia coli, les gènes qui codent pour la résistance aux antibiotiques comprenant la kanamycine, l'ampicilline et le chloramphénicol, et un puissant promoteur artificiel qui contrôle la transcription d'un ARN messager dicistronique, codant pour la résistance à l'ampicilline et au chloramphénicol.

**2.** Vecteur selon la revendication 1, dans lequel l'origine de réplication fonctionnelle que l'on trouve dans Bacillus est celle du plasmide pUB110.

**3.** Vecteur selon la revendication 1, dans lequel l'origine de réplication fonctionnelle que l'on trouve dans Escherichia coli est celle du plasmide pBR322.

**4.** Vecteur selon la revendication 1, dans lequel la séquence nucléotidique du promoteur artificiel, est :

**ATTCATAAATTTGAGAGCTCAAAGGAGGAATTCTTATG 3'**

**TAAGTATTTAAACTCTCGAGTTTCCTCCTTAAGAATACTTAA 5'**

**5.** Vecteur selon la revendication 1, dans lequel la séquence d'ADN code pour une protéine immunologiquement ou biologiquement active.

**6.** Procédé selon la revendication 5, dans lequel la protéine hétérologue est une sous-unité mature de la toxine coquelucheuse.

**7.** Vecteur selon la revendication 1, déposé en tant que <u>Bacillus subtilis</u> SMS 118 (pSM 214) (ATCC 67320).

**8.** Plasmide recombinant, utile pour l'expression d'une protéine hétérologue, obtenu par retrait à partir du vecteur de clonage, défini dans les revendications 1 à 7, du gène de la $\beta$-lactamase, à l'aide des enzymes de restriction EcoRI et HindIII, et clonage des séquences d'ADN codant pour la protéine hétérologue dans les sites de restriction.

**9.** Plasmide recombinant selon la revendication 8, dans lequel la protéine hétérologue est une sous-unité de la toxine coquelucheuse.

**10.** Plasmide recombinant pSM226 (ATCC 67321) selon la revendication 9, dans lequel la séquence d'ADN code pour la sous-unité mature S2.

**11.** Plasmide recombinant pSM228 (ATCC 67322) selon la revendication 9, dans lequel la séquence d'ADN code pour la sous-unité mature S3.

**12.** Plasmide recombinant pSM244 (ATCC 67324) selon la revendication 9, dans lequel la séquence d'ADN code pour la sous-unité mature S4.

**13.** Plasmide recombinant PSM243 (ATCC 67323) selon la revendication 9, dans lequel la séquence d'ADN code pour la sous-unité mature S5.

**14.** Micro-organisme transformé avec un plasmide recombinant défini dans les revendications 8 à 13.

**15.** Micro-organisme selon la revendication 14, choisi parmi <u>Escherichia coli</u> et <u>Bacillus subtilis</u>.

**16.** Micro-organisme selon la revendication 15, qui est <u>Bacillus subtilis</u> SMS 118.

**17.** <u>Bacillus subtilis</u> SMS 118 (pSM226) (ATCC 67321).

**18.** <u>Bacillus subtilis</u> SMS 118 (pSM228) (ATCC 67322).

**19.** <u>Bacillus subtilis</u> SMS 118 (pSM244) (ATCC 67324).

**20.** <u>Bacillus subtilis</u> SMS 118 (pSM243) (ATCC 67323).

**21.** Procédé de préparation de protéines hétérologues, caractérisé en ce qu'on cultive un micro-organisme transformé selon les revendications 14 à 20, dans un milieu liquide contenant des sources d'azote, de carbone et des sels minéraux, en présence de chloramphénicol.

**22.** Procédé selon la revendication 21, dans lequel le micro-organisme est <u>B. subtilis</u>, SMS 118 (pSM226) (ATCC 67321), et la protéine hétérologue est la sous-unité S2 mature.

**23.** Procédé selon la revendication 21, dans lequel le micro-organisme est <u>B. subtilis</u>, SMS 118 (pSM228) (ATCC 67322), et la protéine hétérologue est la sous-unité S3 mature.

**24.** Procédé selon la revendication 21, dans lequel le micro-organisme est <u>B. subtilis</u>, SMS 118 (pSM244) (ATCC 67324), et la protéine hétérologue est la sous-unité S4 mature.

**25.** Procédé selon la revendication 21, dans lequel le micro-organisme est <u>B. subtilis</u>, SMS 118 (pSM243) (ATCC 67323), et la protéine hétérologue est la sous-unité S5 mature.

Figur 1-A

Figure 1-B

Figure 2

24

Figure 3

Figure 4

Figure 5

Figure 6

Figure 7

29

EcoRI    +1 +2 +3 +4 +5 +6 +7 +8
AATTCTTATGATATACAGCCCGGCTGACGTCGCC
TTAAGAATACTATATGTCGGGCCGACTGCAGCGG

Figure 8

Figure 9

Figura 10-A

**A**

Figure 10-B